# EUROPÄISCHE PATENTSCHRIFT

(11) **EP 4 085 881 B1**
(45) Veröffentlichungstag und Bekanntmachung des Hinweises auf die Patenterteilung: **27.11.2024**
(21) Anmeldenummer: 22169388.0
(22) Anmeldetag: 22.04.2022
(51) Int. Cl.: A61F 9/00, B05B 9/08, B05B 9/04, B65D 47/18, A61M 11/00, B05B 11/00, A61D 1/02, A61D 7/00

(54) **VORRICHTUNG ZUR TROPFENWEISEN ABGABE EINES FLUIDS**
DEVICE FOR DISPENSING A FLUID IN DROPS
DISPOSITIF DE DISTRIBUTION GOUTTE À GOUTTE D'UN LIQUIDE

(30) Priorität: 05.05.2021 DE 102021111630
(43) Veröffentlichungstag der Anmeldung: 09.11.2022
(73) Patentinhaber: Henke-Sass, Wolf GmbH, 78532 Tuttlingen (DE)
(72) Erfinder: RIEDLINGER, Axel, 72355 Schömberg (DE); SCHMIDT, Anika, 78567 Fridingen (DE); WIEDMANN, Maikel, 78194 Immendingen (DE)
(74) Vertreter: Patentanwälte Geyer, Fehners & Partner mbB

(56) Entgegenhaltungen:
- WO-A1-2004/028420
- WO-A1-2011/126569
- WO-A1-2012/159026
- US-A- 4 623 337
- US-A- 6 105 828

## Beschreibung

Die vorliegende Erfindung betrifft eine Vorrichtung zur tropfenweisen Abgabe eines Fluids mit den Merkmalen des Oberbegriffs des Anspruches 1.

Wenn es sich bei dem Fluid um z.B. einen Impfstoff handelt, kann mit einer solchen Vorrichtung eine tropfenweise Abgabe des Impfstoffes durchgeführt werden.

Wünschenswert ist beispielsweise eine Abgabe von Augentropfen an Geflügel zur Impfung, um somit eine okulare Impfung durchzuführen. Bisher wird eine solche okulare Impfung mit Hilfe einer Kunststoffflasche durchgeführt. Durch händischen Druck auf die Flaschenwand wird ein Tropfen an einem Auslassaufsatz erzeugt. Sobald sich der Tropfen gelöst hat, wird der Druck entfernt, so dass der restliche Impfstoff durch den entstandenen Unterdruck zurück in die Flasche gezogen wird. Tropfengröße und Tropfenanzahl sind sowohl abhängig vom Druck, der vom Anwender ausgeübt wird, als auch vom Winkel, mit dem die Flasche bei der Tropfenabgabe gehalten wird.

Bei der praktischen Anwendung zur okularen Impfung eines Tieres kann es beispielsweise vorkommen, dass sich zwei oder mehr Tropfen lösen, da in der Regel eine große Anzahl von Tieren unter hohem Zeitdruck versorgt werden müssen. Es liegt somit eine anwenderbezogene Varianz vor, die einerseits zu Schwankungen bei der Dosierung und andererseits zu einem schwer kalkulierbaren Bedarf an Impfstoff für einen quantitativ definierten Tierbestand führt.

Die WO 2012/159026 A1 und die WO 2011/126569 A1 zeigen jeweils eine Vorrichtung zur tropfenweisen Abgabe eines Fluids der eingangs genannten Art. Die US 4 623 337 A zeigt eine Vorrichtung zur tropfenweisen Abgabe eines Fluids, die keine Pumpe aufweist, sondern manuell zu betätigen ist.

Ausgehend hiervon soll eine Vorrichtung zur tropfenweisen Abgabe eines Fluids der eingangs genannten Art so weitergebildet werden, dass die beschriebenen Schwierigkeiten möglichst vollständig überwunden werden können.

Die Erfindung ist im Anspruch 1 definiert. Vorteilhafte Weiterbildungen sind in den abhängigen Ansprüchen angegeben.

Aufgrund der mittels des Betätigungselements betätigbaren Pumpe kann stets eine definierte Fluidmenge zur Austrittsöffnung gefördert werden, so dass die Tropfengröße und Tropfenanzahl gewährleistet werden kann. Die Tropfenbildung wird insbesondere dadurch gewährleistet, dass sich der Fluidkanal in Richtung zur Austrittsfläche verjüngt, da die zweite Querschnittsfläche kleiner ist als die erste Querschnittsfläche.

Die Vorrichtung kann insbesondere so ausgebildet werden, dass eine Betätigung des Betätigungselements, das beispielsweise als Druckknopf realisiert sein kann, eine definierte Laufzeit der Pumpe bewirkt, so dass dadurch eine definierte Fluidmenge zur Austrittsöffnung gepumpt wird. Es können mehrere Pumpzyklen der Pumpe notwendig sein, um die Fluidmenge für einen Tropfen zu fördern. Wenn es sich bei dem Fluid um ein Medikament oder einen Impfstoff handelt, kann beispielsweise eine Tropfengröße von 0,03 ml gewünscht sein. Dafür können mehrere Pumpzyklen (beispielsweise vier) notwendig sein. Bei der Pumpe kann es sich beispielsweise um eine Membranpumpe handeln.

Die Vorrichtung kann ein Eingabemodul (beispielsweise mit einer Anzeige und einem oder mehreren Eingabeelementen) aufweisen, über das z.B. die Tropfengröße und/oder die Tropfenanzahl einstellbar ist, die bei einmaliger Betätigung des Betätigungselementes erzeugt werden.

Bevorzugt ist die Vorrichtung so ausgebildet, dass bei einmaliger Betätigung des Betätigungselementes genau ein einziger Tropfen gebildet und abgegeben wird.

Die Vorrichtung kann einen Zähler aufweisen, der die Anzahl der insgesamt abgegebenen Tropfen zählt. Diese Anzahl kann z.B. auf der Anzeige dargestellt werden.

Ferner kann die Vorrichtung eine Temperaturmesseinrichtung zur Messung der Temperatur des zu fördernden Fluids aufweisen. Dabei kann es sich um einen Temperatursensor handeln, der beispielsweise das Fluid in der Fluidverbindung zur Pumpe oder das Fluid in der Fluidverbindung von der Pumpe bis zum Fluidanschluss (bevorzugt an einer definierten Stelle) misst. Die gemessene Temperatur kann z.B. auf der Anzeige angezeigt werden, um den Anwender zu informieren, und/oder kann zur Ausgabe einer Warnung genutzt werden, wenn ein definierbarer Schwellwert über- oder unterschritten wird oder wenn die gemessene Temperatur außerhalb eines definierbaren Intervalls liegt.

Die gemessene Temperatur kann einer Steuereinheit der Vorrichtung zur tropfenweisen Abgabe eines Fluids zugeführt werden, die in Abhängigkeit der Temperatur z.B. die Motorlaufzeit bei Betätigung des Betätigungselementes anpasst, um zu gewährleisten, dass die gleiche Fluidmenge gefördert bzw. gepumpt wird. So kann beispielsweise mit steigender Temperatur die Viskosität des Fluids zunehmen, was zu einer längeren Pumplaufzeit führt, um dieselbe Fluidmenge zu fördern.

Die gemessene Temperatur kann ferner in einem Kalibriermodus der Vorrichtung zur Anpassung der Pumplaufzeit genutzt werden.

Ferner kann der Fluidkanal einen an den zweiten Kanalabschnitt anschließenden dritten Kanalabschnitt aufweisen, der sich bis zur Austrittsöffnung erstreckt und eine dritte Querschnittsfläche aufweist, die größer ist als die zweite Querschnittsfläche. Dies fördert die Tropfenbildung. Des Weiteren kann der dritte Kanalabschnitt insbesondere zylinderförmig ausgebildet sein, wobei sein Durchmesser an die gewünschte Tropfengröße angepasst ist.

So kann die Austrittsdüse eine auswechselbare Endkappe mit dem dritten Kanalabschnitt aufweisen, so dass verschiedene Endkappen mit verschiedenen Durchmessern oder Querschnittsflächen des dritten Kanalabschnitts bereitgestellt werden können, von denen jeweils die Endkappe gewählt wird, die den gewünschten Durchmesser (bzw. die gewünschte Querschnittsfläche) des dritten Kanalabschnitts für die geforderte Tropfengröße aufweist.

Die Austrittsdüse kann an der Austrittsöffnung einen ersten Bereich mit einem ersten Außendurchmesser und einen sich daran in der Richtung entgegen der Fluidrichtung anschließenden zweiten Bereich mit einem zweiten Außendurchmesser aufweisen, wobei der zweite Außendurchmesser kleiner ist als der erste Außendurchmesser. Der erste Bereich endet bevorzugt am distalen Ende der Austrittsdüse. Es liegt somit ein äußerer Einstich vor, der vorteilhaft für den gewünschten Tropfenabriss an der Austrittsöffnung ist.

Ferner kann die Innenkante der Austrittsöffnung scharfkantig ausgebildet sein. Des Weiteren kann die Außenkante an der Austrittsöffnung scharfkantig ausgebildet sein. Unter scharfkantig wird hier insbesondere verstanden, dass keine Fase ausgebildet ist und/oder dass die an der jeweiligen Kante zusammenstoßenden Materialgrenzflächen einen Winkel aus dem Bereich von 80° bis 100° und bevorzugt aus dem Bereich von 85° bis 95° und insbesondere bevorzugt einen Winkel von 90° einschließen.

Für einen Tropfen mit 0,03 ml weist der dritte Kanalabschnitt, der bevorzugt einen kreisförmigen Querschnitt aufweist, einen Durchmesser von beispielsweise 1,5 mm auf. Der Durchmesser kann im Bereich von 0,5 mm bis 3 mm (bevorzugt bis 2 mm) liegen. Die Querschnittsfläche des dritten Kanalabschnitts kann im Bereich von 0,20 bis 7,07 mm² liegen. Mit steigender Tropfengröße nimmt der optimale Durchmesser des dritten Kanalabschnittes zu.

Der erste Kanalabschnitt, der bevorzugt einen kreisförmigen Querschnitt aufweist, kann einen Durchmesser von beispielsweise 3 bis 6 mm aufweisen. Die Querschnittsfläche des ersten Kanalabschnitts kann im Bereich von 7,07 bis 28,27 mm² liegen.

Der zweite Kanalabschnitt kann einen, zwei oder mehr zweite Kanalteilabschnitte aufweisen, die z.B. einen kreissegmentförmigen Querschnitt aufweisen. Die Querschnittsfläche des zweiten Kanalabschnitts kann im Bereich von 0,05 bis 1 mm² und bevorzugt im Bereich von 0,1 bis 0,5 mm² und besonders bevorzugt im Bereich von 0,15 bis 0,30 mm² liegen.

Die dritte Querschnittsfläche kann insbesondere kleiner als die erste Querschnittsfläche sein.

Der zweite Kanalabschnitt schließt bevorzugt direkt an den ersten Kanalabschnitt an. Ferner schließt der dritte Kanalabschnitt bevorzugt direkt an den zweiten Kanalabschnitt an.

Mit der erfindungsgemäßen Vorrichtung können insbesondere Tropfen mit einem Volumen von 0,025 ml bis 0,04 ml erzeugt werden.

Es können beispielsweise 10, 20, 30, 40, 50, 60, 70, 80, 90 oder 100 Tropfen pro Minute erzeugt werden. Bei der Erzeugung von genau einem Tropfen pro Betätigung des Betätigungselementes wird diese Zahl im Wesentlichen dadurch limitiert, wie schnell das Betätigungselement wiederholt betätigt werden kann.

Es hat sich gezeigt, dass eine verbesserte Tropfenbildung erreicht werden kann, wenn der Fluidkanal der Austrittsdüse so ausgebildet ist, dass ein geradliniges Durchströmen verhindert ist.

Ferner ist es von Vorteil, wenn in dem Fluidkanal der Austrittsdüse ein Rückschlagventil angeordnet ist, das ab einem vorbestimmten Druck des von der Pumpe kommenden Fluids öffnet und so eine Fluidverbindung der Pumpe mit der Austrittsöffnung herstellt. Dadurch kann ein Abbremsen der Geschwindigkeit des gepumpten Fluids erzeugt werden, was wiederum zur Tropfenbildung beiträgt und insbesondere verhindert, dass das Fluid als Strahl abgegeben wird. Dieses Rückschlagventil dient somit hauptsächlich dazu, die Tropfenbildung zu gewährleisten.

Die Vorrichtung weist einen Neigungssensor auf, der die aktuelle Neigung der Vorrichtung und somit der Austrittsdüse zur Erdoberfläche misst und an die Steuereinheit abgibt. Die Vorrichtung ist ferner so ausgelegt, dass ein Betätigen des Betätigungselements nur dann die Pumpe aktiviert, wenn die gemessene Neigung innerhalb eines vorbestimmten Neigungsbereiches liegt. Dabei kann es sich beispielsweise um den Neigungsbereich von 30 bis 40° handeln. Wenn ein Benutzer die Vorrichtung in dieser Neigung hält, kann er durch Betätigung des Betätigungselements die gewünschte Anzahl von Tropfen abgeben. Die Vorrichtung kann so ausgebildet sein, dass sie dem Benutzer akustisch, haptisch, und/oder optisch mitteilt, dass er die Vorrichtung so hält, dass die Neigung innerhalb des vorbestimmten Neigungsbereiches liegt oder nicht liegt.

Für die Einstellung des Volumens kann die Steuereinheit detektieren, ab wann sich ein Motor der Pumpe zu drehen beginnt, nachdem die Pumpe durch Betätigen des Betätigungselementes aktiviert wurde. Dieser Zeitpunkt ist nicht bei jeder Betätigung des Betätigungselementes gleich, sondern hängt von äußeren Einflussfaktoren ab, wie z.B. der Widerstand, gegen den der Motor anlaufen muss. Ab diesem Zeitpunkt wird die Pumpe für eine definierte Zeitspanne mit Spannung versorgt und am Ende der Zeitspanne wird der Motor der Pumpe aktiv gebremst. Dadurch wird erreicht, dass die undefinierbaren Einflüsse von Motoranlaufzeit und Motorauslaufzeit minimiert werden. Ein aktives Abbremsen kann z.B. dadurch erreicht werden, dass die Laufrichtung des Motors am Ende der Zeitspanne umgekehrt wird, so dass der Motor versucht, die Motorwelle in die entgegengesetzte Richtung zu drehen.

Die erfindungsgemäße Vorrichtung wird bevorzugt kalibriert. Dabei kann der Anwender die Kalibrierung mit einfachen Mitteln durchführen. Der Anwender kann dazu einen Kalibrierungsmodus über die Eingabeeinheit auswählen, wobei ein bestimmtes Zielvolumen für z.B. ein bestimmtes Medikament vorgegeben ist. Alternativ kann der Anwender ein Zielvolumen auswählen oder vorgeben. Sobald dies abgeschlossen ist, betätigt der Anwender das Betätigungselement so oft, bis er in ein Gefäß das definierte Zielvolumen abgegeben hat. Über die Anzahl der benötigten Betätigungen bis zum Erreichen des Zielvolumens ermittelt die Steuereinheit der Vorrichtung die optimale aktive Pumpenlaufzeit. Durch diese Art der Kalibrierung können äußere Einflüsse auf das Förderverhalten der Pumpe ausgeglichen werden. Dabei kann es sich z.B. um die Art des Fluids, die Temperatur des Fluids sowie die Vorspannung des Rückschlagventils im Fluidkanal der Austrittsdüse handeln. Gerade die Vorspannung des Rückschlagventils variiert vor allem aufgrund von nicht zu vermeidenden Fertigungstoleranzen.

Bei dem abzugebenden Fluid kann es sich insbesondere um eine Flüssigkeit handeln. Bevorzugt kann das Fluid ein Medikament, ein Wirkstoff oder ein Impfstoff sein. Die tropfenweise Abgabe mittels der Vorrichtung kann zur Abgabe in ein Auge, eine Körperöffnung, oder auf die Haut eines Tieres (beispielsweise von Geflügel) eingesetzt werden.

Mit dem Fluidanschluss kann ein Reservoir des Fluids verbunden sein. Dabei kann es sich beispielsweise um einen Behälter mit dem Fluid, wie z.B. eine Medikamentenflasche handeln. Es ist jedoch auch eine Schlauchverbindung zu einem stationären Reservoir möglich.

Die erfindungsgemäße Vorrichtung ist insbesondere als tragbares Gerät ausgebildet. Die erfindungsgemäße Vorrichtung kann ein Gehäuse mit einem Griffabschnitt aufweisen, der bevorzugt mit einer Hand gehalten werden kann. Am Griffabschnitt kann das Betätigungselement vorgesehen sein. Insbesondere kann es so vorgesehen sein, dass ein Benutzer mit dem Zeigefinger seiner den Griffabschnitt haltenden Hand das Betätigungselement bedienen kann.

Es wird ferner ein Verfahren zur tropfenweisen Abgabe eines Fluids bereitgestellt, wobei eine Vorrichtung mit einem Fluidanschluss, über den das abzugebende Fluid zuführbar ist, einer Pumpe, die in Fluidverbindung mit dem Fluidanschluss steht, einer Austrittsdüse, die in Fluidverbindung mit der Pumpe steht, und einem Betätigungselement, das bei Betätigung die Pumpe aktiviert, die zugeführtes Fluid zur Austrittsdüse zur tropfenweisen Abgabe pumpt, verwendet wird, wobei die Austrittsdüse eine Austrittsöffnung und einen sich bis zur Austrittsöffnung erstreckenden Fluidkanal aufweist, der in Fluidrichtung in der Pumpe bis zur Austrittsöffnung einen ersten Kanalabschnitt mit einer ersten Querschnittsfläche und einen an den ersten Kanalabschnitt anschließenden zweiten Kanalabschnitt mit einer zweiten Querschnittsfläche aufweist, wobei die zweite Querschnittsfläche kleiner ist als die erste Querschnittsfläche,
wobei zur Abgabe eines Tropfens das Betätigungselement betätigt wird.

Das Verfahren kann in gleicher Weise weitergebildet werden, wie die erfindungsgemäße Vorrichtung.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbespiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine isometrische Ansicht eines Ausführungsbeispiels der erfindungsgemäßen Vorrichtung 1 zur Abgabe einer tropfenweisen Abgabe eines Fluids;
- Fig. 2: eine Schnittansicht der Vorrichtung 1 von Fig. 1;
- Fig. 3 und 4: schematische Darstellungen zur Erläuterung der Funktionsweise einer Membranpumpe;
- Fig. 5: eine vergrößerte Schnittansicht des Details A von Fig. 2 und
- Fig. 6: eine Schnittansicht entlang der Schnittlinie B-B in Fig. 5.

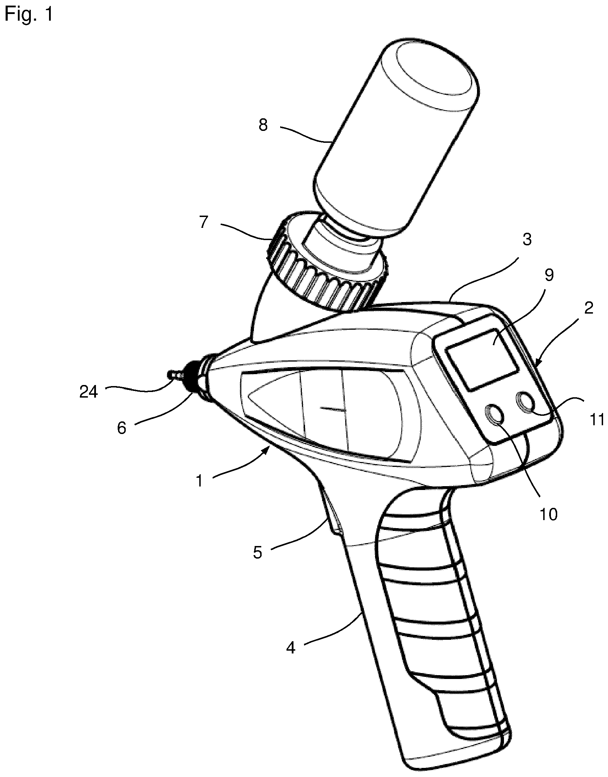

Bei dem in Fig. 1 gezeigten Ausführungsbeispiel umfasst die erfindungsgemäße Vorrichtung 1 zur tropfenweisen Abgabe eines Fluids in ein Auge ein Gehäuse 2 mit einem Hauptabschnitt 3 und einem Griffabschnitt 4. Der Griffabschnitt 4 ist so ausgebildet, dass ein Benutzer die Vorrichtung 1 durch Umgreifen des Griffabschnittes 4 mit einer Hand halten kann. Des Weiteren weist der Griffabschnitt 4 einen Startknopf 5 zum Betätigen der Vorrichtung 1 auf. Am vorderen Ende des Hauptabschnitts 3 ist ein Abgabebereich 6 ausgebildet. Des Weiteren weist die Vorrichtung 1 am oberen Bereich des Hauptabschnitts 3 einen Anschluss 7 auf, an dem in der in Fig. 1 gezeigten Ausführungsform ein Behälter 8 mit dem abzugebenden Fluid (hier in Form einer Medikamentenflasche) befestigt ist.

Am hinteren Ende des Hauptabschnittes 3 sind eine Anzeige 9 sowie zwei Bedienelemente 10, 11 vorgesehen.

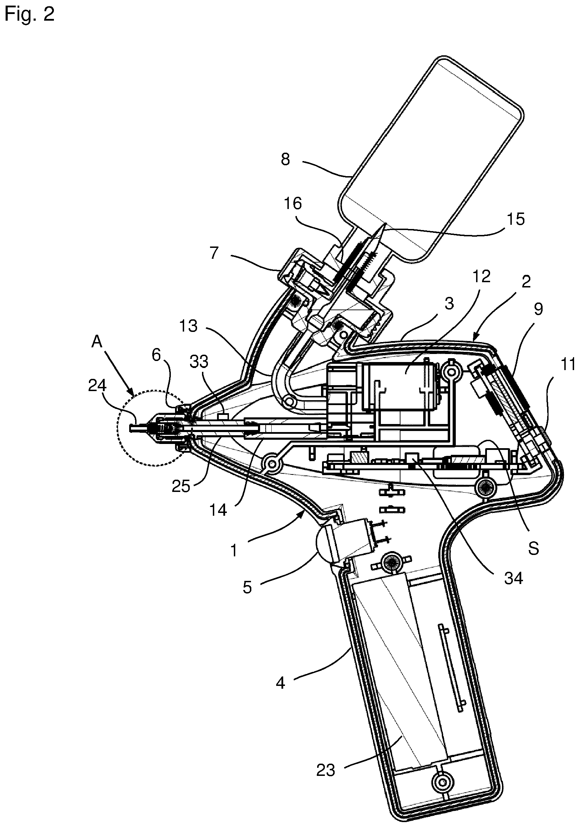

Wie am Besten in der Schnittansicht in Fig. 2 ersichtlich ist, ist im Hauptabschnitt 3 eine Pumpe 12 vorgesehen, die über eine erste Fluidverbindung 13 mit dem Anschluss 7 und über eine zweite Fluidverbindung 14 mit dem Abgabebereich 6 verbunden ist. Für die beiden Fluidverbindungen 12 und 14 werden hier Schläuche verwendet.

Der Anschluss 7 weist einen Einstichdorn 15 auf, mit dem beim Fixieren des Behälters 8 eine Membran 16 am Behälter 8 durchstochen wird, um so eine Verbindung für das Fluid vom Inneren des Behälters 8 zur ersten Fluidverbindung 13 zu gewährleisten. Der Einstichdorn 15 umfasst ferner ein Ventil, das für die automatische Entlüftung des Behälters 8 und somit für den notwendigen Druckausgleich sorgt.

Bei der Pumpe 12 kann es sich beispielsweise um eine Membranpumpe 12 handeln, die für Mikro-Flussraten ausgelegt ist, da die abzugebenden Tropfen z.B. ein Volumen von ca. 0,03 ml aufweisen sollen.

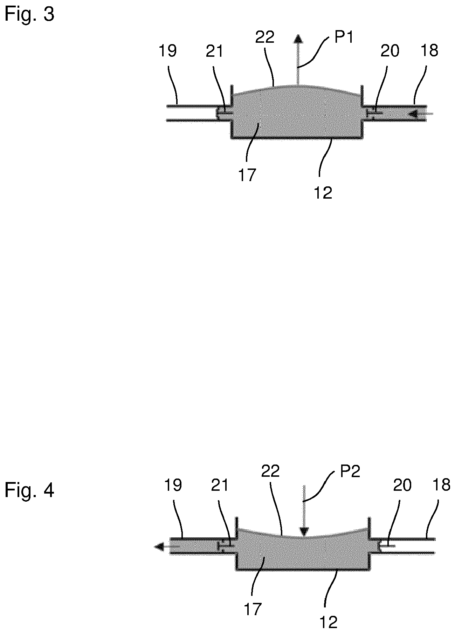

Das grundlegende Prinzip einer solchen Membranpumpe 12 wird unter Bezugnahme auf die schematische Darstellung in den Fig. 3 und 4 näher erläutert. Die Pumpe 12 umfasst eine Pumpenkammer 17 mit einem Pumpeneingang 18 und einem Pumpenausgang 19, die jeweils mit einem Rückschlagventil 20, 21 verschlossen sind. Die Rückschlagventile 20, 21 sind so angeordnet, dass nur ein Fluss des zu pumpenden Fluids vom Pumpeneingang 18 in die Pumpenkammer 17 und von dort zum Pumpenausgang 19 möglich ist. Die Pumpenkammer 17 ist an einer Seite durch eine Membran 22, die beispielsweise aus einem flexiblen Elastomer gebildet ist, begrenzt. Die Membran 22 kann von der Pumpe 12 durch einen Motor (nicht gezeigt) über einen Exzenter (nicht gezeigt) so in Schwingung versetzt werden, dass durch eine Bewegung der Membran 22 nach oben (Pfeil P1 in Fig. 3) das Volumen der Pumpenkammer 17 vergrößert und durch eine Bewegung der Membran 22 nach unten (Pfeil P2 in Fig. 4) das Volumen der Pumpenkammer 17 verringert wird.

Bei der Vergrößerung des Volumens der Pumpenkammer 17 gemäß Fig. 3 wird in der Pumpenkammer 17 ein Unterdruck erzeugt, wodurch Fluid über den Pumpeneingang 18 in die Pumpenkammer 17 gesaugt wird. Bei der Verringerung des Volumens der Pumpenkammer 17 (Fig. 4) wird ein Überdruck erzeugt, wodurch Fluid aus der Pumpenkammer 17 zum Pumpenausgang 19 gedrückt wird.

Ferner ist eine Steuereinheit S (Fig. 2) vorgesehen, die z.B. einen Prozessor und einen Speicher aufweist. Die Steuereinheit S dient zur Steuerung der Pumpe 12, ist mit dem Startknopf 5 und den Bedienelementen 10, 11 verbunden und erzeugt die Daten und Informationen, die auf der Anzeige 9 dargestellt werden.

Da bei der erfindungsgemäßen Vorrichtung 1 die erste Fluidverbindung 13 mit dem Pumpeneingang 18 und die zweite Fluidverbindung 14 mit dem Pumpenausgang 19 verbunden ist, kann durch Betätigung der Pumpe 12 Fluid von dem Behälter 8 zum Abgabebereich 6 transportiert werden.

Die Aktivierung der Pumpe 12 erfolgt über die Betätigung des Startknopfes 5, wobei nach Betätigung des Startknopfes 5 aufgrund der Steuerung mittels der Steuereinheit S eine vorbestimmte Anzahl von Pumpzyklen durchgeführt wird, um das notwendige Volumen für mindestens einen abzugebenden Tropfen zu fördern. Zur Energieversorgung der Pumpe 12 und der Steuereinheit S ist im Griffabschnitt 4 eine Energiequelle 23 bzw. eine Stromversorgung 23 (hier z.B. ein Akku oder eine Batterie) vorgesehen.

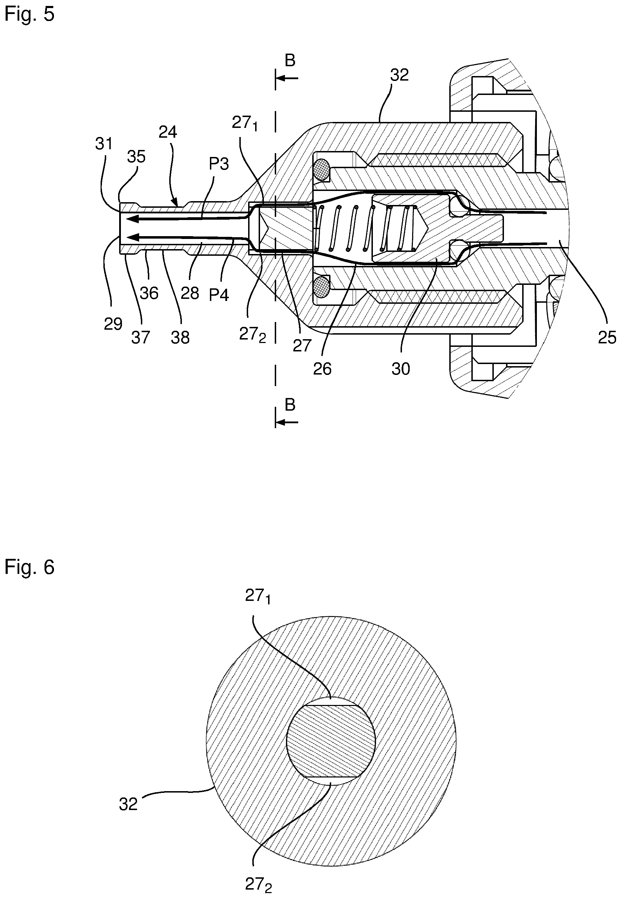

Um zu verhindern, dass das von der Pumpe 12 geförderte Fluidvolumen als Strahl am Abgabebereich 6 der Vorrichtung 1 abgegeben wird, ist die in der vergrößerten Schnittansicht in Fig. 5 gezeigte Austrittsdüse 24 im Abgabebereich 6 vorgesehen. Die Austrittsdüse 24 weist einen Eingangsabschnitt 25, der mit der zweiten Fluidverbindung 14 verbunden ist, einen ersten, zweiten und dritten Kanalabschnitt 26, 27 und 28, eine Austrittsöffnung 29 sowie ein Rückschlagventil 30 auf.

In der in Fig. 5 gezeigten Stellung des Rückschlagventils 30 verschließt dieses den Eingangsabschnitt 25.

Ab Anliegen eines vorbestimmten Druckes (hier z.B. 250 mbar) des von der Pumpe 12 geförderten Fluids wird das Rückschlagventil 30 zur Austrittsöffnung 29 hin bewegt, so dass es öffnet und das Fluid, wie durch die Pfeile P3 und P4 angedeutet ist, durch die Kanalabschnitte 26 bis 28 strömen kann.

Wie der Schnittdarstellung der Austrittsdüse 24 in Fig. 5 sowie der Schnittdarstellung in Fig. 6 ferner entnommen werden kann, ist die Querschnittsfläche des zweiten Kanalabschnittes 27 kleiner als die Querschnittsfläche des ersten Kanalabschnitts 26. Der zweite Kanalabschnitt 27 ist durch zwei Kanalteilabschnitte 27₁ und 27₂ gebildet, die jeweils eine kreisegmentförmigen Querschnitt aufweisen, und weist bei der hier beschriebenen Ausführungsform eine Querschnittsfläche von 0,17 mm² auf, wohingegen der Querschnitt des ersten Kanalabschnitts 26 kreisförmig mit einer Fläche von 10,18 mm² ist. Ferner ist die Querschnittsfläche des zweiten Kanalabschnitts 27 auch kleiner als die Querschnittsfläche des dritten Kanalabschnittes 28, wobei der Querschnitt des dritten Kanalabschnitts 28 kreisförmig mit einer Fläche von 1,77 mm² ist. Durch diese Reduktion der Querschnittsfläche in Verbindung mit dem Rückschlagventil 30 wird eine Abbremsung des Fluidflusses, der von der Pumpe 12 erzeugt wird, erreicht, wodurch das Fluid in Tropfenform über die Austrittsöffnung 29 (nicht als Fluidstrahl) abgegeben wird. Damit kann z.B. die tropfenweise Abgabe des Fluids in ein Auge (beispielsweise ein Tierauge) erfolgen. Eine solche tropfenweise Abgabe kann z.B. zur Impfung von Tieren und somit zu einer okularen Impfung genutzt werden. In dieser Art und Weise kann z.B. eine okulare Impfung an Geflügel durchgeführt werden. Die erfindungsgemäße Vorrichtung 1 kann daher auch als elektronisches Handgerät 1 zur okularen Impfung bezeichnet werden.

Des Weiteren weist die Austrittsöffnung 29 eine Kantenform der Innenkante 31 auf, die für den gewünschten Tropfenabriss vorteilhaft ist und dadurch ein Hängenbleiben des Tropfens an der Austrittsdüse 24 verhindert. Insbesondere ist die Kantenform der Innenkante 31 so gewählt, dass sie möglichst scharfkantig ist und z.B. keine Fase am distalen Ende der Austrittsöffnung 29 vorgesehen ist. Diese Scharfkantigkeit der Austrittsöffnung 29 fördert den Tropfenabriss, da dadurch die Oberfläche minimiert und das Fluid (bzw. die Flüssigkeit) weniger gut an der Austrittsdüse haftet. Neben der scharfkantigen Ausbildung der Innenkante (des Innenrandes) 31 der Austrittsöffnung 29 kann auch die Außenkante 35 am distalen Ende scharfkantig (z.B. ohne Fase) ausgebildet sein. Auch diese fördert den Tropfenabriss. Unter "scharfkantig" wird hier insbesondere verstanden, dass die an der jeweiligen Kante zusammenstoßenden Materialgrenzflächen einen Winkel im Bereich von 80° bis 100° und bevorzugt im Bereich von 85° bis 95° und besonders bevorzugt von 90° einschließen. Ferner ist die Außenkante 35 bevorzugt ohne Fase ausgebildet.

Zusätzlich ist ein äußerer Einstich 36 (Verringerung des Außendurchmessers am distalen Ende im Bereich des dritten Kanalabschnitts 28) vorgesehen, der verhindert, dass das Fluid bzw. die Flüssigkeit an der Austrittsdüse 24 entlangläuft, was zu einer unerwünschten Vergrößerung der Kontaktfläche führen würde. Der äußerer Einstich 36 ist auch für den gewünschten Tropfenabriss vorteilhaft. Durch den Einstich 36 weist die Austrittsdüse 24 an der Austrittsöffnung 29 einen ersten Bereich 37 mit einem ersten Durchmesser und einen sich an den ersten Bereich 37 in Richtung entgegen der Fluidrichtung anschließenden zweiten Bereich 38 mit einem zweiten Außendurchmesser auf, wobei der zweite Außendurchmesser kleiner ist als der erste Außendurchmesser.

Die Austrittsdüse 24 kann, wie in Fig. 5 gezeigt ist, eine auswechselbare Endkappe 32 aufweisen, die den dritten Kanalabschnitt 28 umfasst. Dazu kann die Endkappe 32 beispielsweise schraubbar ausgebildet sein.

Da der Innendurchmesser bzw. die Querschnittsfläche des dritten Kanalabschnitts 28 die Tropfengröße beeinflusst, können mehrere Endkappen 32 mit unterschiedlichen dritten Kanalabschnitten 28, die sich in ihrer Querschnittsfläche bzw. im Innendurchmesser unterscheiden, vorgesehen sein. Durch Auswahl der jeweils geeigneten Endkappe 32 kann somit die gewünschte Tropfengröße mit eingestellt werden.

Ferner kann die Vorrichtung 1 einen Temperatursensor 33 (Fig. 2) aufweisen, der die Temperatur des Fluids kurz vor der Austrittsdüse 24 misst. Das Messsignal bzw. die Messgröße wird der Steuereinheit S mitgeteilt, da der Temperatursensor 33 mit der Steuereinheit S verbunden ist. In Abhängigkeit der gemessenen Temperatur kann dann die Steuereinheit S, sofern dies notwendig ist, Änderungen bei der Ansteuerung der Pumpe 12 nach Betätigung des Startknopfes 5 durchgeführt werden. So ändert sich beispielsweise die Viskosität von Medikamenten und Impfstoffen mit der Temperatur, was beispielsweise durch längere oder kürzere Pumpzeiten kompensiert werden kann, um immer noch gleich große Tropfen zu erzeugen. Auch kann es notwendig sein, dass erst ab einer gewissen Temperatur des Fluids eine tropfenweise Abgabe gewünscht ist. In diesem Fall kann z.B. eine Abgabe unter der vorbestimmten Temperatur durch die Steuereinheit S verhindert werden und/oder die gemessene Fluidtemperatur kann auf der Anzeige 9 dargestellt werden.

Ferner kann die Vorrichtung 1 einen Neigungssensor 34 (Fig. 2) aufweisen, der ebenfalls mit der Steuereinheit S verbunden ist. Der Neigungssensor 34 misst die Neigung der Vorrichtung und somit der Austrittsdüse 24 relativ zur Erdoberfläche. Die Steuereinheit S kann so ausgelegt sein, dass eine Aktivierung der Pumpe 12 nach Betätigung des Startknopfes 5 nur dann erfolgt, wenn die in diesem Moment gemessene Neigung der Vorrichtung 1 einen vorbestimmten Wert aufweist oder in einem vorbestimmten Bereich liegt. So kann es beispielsweise sein, dass die Vorrichtung 1 für eine Tropfenabgabe bei einer Neigung von 35° ausgelegt ist. In diesem Fall kann die Steuereinheit beispielsweise so ausgelegt sein, dass eine Abgabe nur dann möglich ist, wenn die gemessene Neigung nicht mehr als ± 1°, ± 2°, ± 3°, ± 4°, ± 5° oder ± 6° von der vorgegebenen Neigung (hier 35°) abweicht. Auch dies dient dazu, eine reproduzierbare Tropfengröße zu gewährleisten.

Die Vorrichtung 1 kann so ausgelegt sein, dass dem Benutzer akustisch, haptisch und/oder optisch mitgeteilt wird, ob die Neigung der Vorrichtung 1 im zulässigen Bereich oder außerhalb des zulässigen Bereiches ist.

## Patentansprüche

1. Vorrichtung zur tropfenweisen Abgabe eines Fluids,
wobei die Vorrichtung
einen Fluidanschluss (7), über den das abzugebende Fluid zuführbar ist,
eine Pumpe (12), die in Fluidverbindung mit dem Fluidanschluss (7) steht,
eine Austrittsdüse (24), die in Fluidverbindung mit der Pumpe (12) steht,
ein Betätigungselement (5), und
eine Steuereinheit (S), die bei Betätigung des Betätigungselementes (5) die Pumpe (12) aktiviert, die zugeführtes Fluid zur Austrittsdüse (24) zur tropfenweisen Abgabe pumpt, aufweist,
wobei die Austrittsdüse (24) eine Austrittsöffnung (29) und einen sich bis zur Austrittsöffnung erstreckenden Fluidkanal aufweist, der in Fluidrichtung von der Pumpe (12) bis zur Austrittsöffnung (29) einen ersten Kanalabschnitt (26) mit einer ersten Querschnittsfläche und einen an den ersten Kanalabschnitt (26) anschließenden zweiten Kanalabschnitt (27) mit einer zweiten Querschnittsfläche aufweist,
wobei die zweite Querschnittsfläche kleiner ist als die erste Querschnittsfläche, **dadurch gekennzeichnet, dass**
ein Neigungssensor (34) vorgesehen ist, der die Neigung der Vorrichtung (1) zur Erdoberfläche misst und an die Steuereinheit (S) weiterleitet, wobei die Steuereinheit (S) die Pumpe (12) nach Betätigung des Betätigungselementes (5) nur dann aktiviert, wenn die gemessene Neigung innerhalb eines vorbestimmten Neigungsbereiches liegt.

2. Vorrichtung nach Anspruch 1, wobei
der Fluidkanal einen an den zweiten Kanalabschnitt (27) anschließenden dritten Kanalabschnitt (28) aufweist, der sich bis zur Austrittsöffnung (29) erstreckt und eine dritte Querschnittsfläche aufweist, die größer ist als die zweite Querschnittsfläche.

3. Vorrichtung nach Anspruch 2, wobei
sich der dritte Kanalabschnitt (28) geradlinig erstreckt.

4. Vorrichtung nach Anspruch 2 oder 3, wobei
die Austrittsdüse (24) eine auswechselbare Endkappe (32) aufweist, in der der dritte Kanalabschnitt (28) ausgebildet ist.

5. Vorrichtung nach einem der Ansprüche 2 bis 4, wobei
die Austrittsdüse (24) an der Austrittsöffnung (29) einen ersten Bereich (37) mit einem ersten Außendurchmesser aufweist, wobei sich an den ersten Bereich (35) in Richtung entgegen der Fluidrichtung ein zweiter Bereich (38) mit einem zweiten Durchmesser anschließt, wobei der zweite Durchmesser geringer ist als der erste Durchmesser.

6. Vorrichtung nach einem der obigen Ansprüche, wobei
die dritte Querschnittsfläche des dritten Kanalabschnitts (28) kleiner ist als die erste Querschnittsfläche des ersten Kanalabschnitts (26).

7. Vorrichtung nach einem der obigen Ansprüche, wobei
die Innenkante (31) der Austrittsöffnung (29) scharfkantig ausgebildet ist.

8. Vorrichtung nach einem der obigen Ansprüche, wobei
der Fluidkanal der Austrittsdüse (24) so ausgebildet ist, dass ein geradliniges Durchströmen verhindert ist.

9. Vorrichtung nach einem der obigen Ansprüche, wobei
im Fluidkanal der Austrittsdüse (24) ein Rückschlagventil (30) angeordnet ist, das ab einem vorbestimmten Druck des von der Pumpe (12) kommenden Fluids öffnet und so eine Fluidverbindung der Pumpe (12) mit der Austrittsöffnung (29) herstellt.

10. Vorrichtung nach einem der obigen Ansprüche, wobei
die Pumpe (12) als Membranpumpe ausgebildet ist

11. Vorrichtung nach einem der obigen Ansprüche, wobei
mehrere Pumpzyklen der Pumpe (12) notwendig sind, um das Fluidvolumen für einen abzugebenden Tropfen zu fördern.

12. Vorrichtung nach einem der obigen Ansprüche, wobei
eine Temperaturmesseinrichtung (33) vorgesehen ist, die die Temperatur des abzugebenden Fluids misst und an die Steuereinheit (S) weiterleitet, die in Abhängigkeit der gemessenen Temperatur die Ansteuerung der Pumpe (12) variiert, um eine temperaturbedingte Volumenänderung des geförderten Volumens zu kompensieren.

13. Vorrichtung nach einem der obigen Ansprüche, wobei
die Pumpe (12) einen Motor aufweist und die Steuereinrichtung (S) bei Aktivierung der Pumpe (12) detektiert, wann sich der Motor der Pumpe (12) zu drehen beginnt und ab diesem Zeitpunkt nach Ablauf der definierten Zeitspanne für den Betrieb der Pumpe (12) den Motor der Pumpe (12) aktiv bremst.

## Claims

1. A device for dispensing a fluid drop-by-drop, wherein the device has
a fluid connector (7), the fluid to be dispensed being able to be supplied thereby,
a pump (12) which is fluidically connected to the fluid connector (7),
an outlet nozzle (24) which is fluidically connected to the pump (12),
an actuating element (5), and
a control unit (S) which, when the actuating element (5) is actuated, activates the pump (12) which pumps the supplied fluid to the outlet nozzle (24) for dispensing drop-by-drop,
wherein the outlet nozzle (24) has an outlet opening (29) and a fluid channel which extends as far as the outlet opening and which, in the direction of the fluid from the pump (12) as far as the outlet opening (29), has a first channel portion (26) with a first cross-sectional surface and a second channel portion (27) with a second cross-sectional surface adjoining the first channel portion (26), wherein the second cross-sectional surface is smaller than the first cross-sectional surface, **characterized in that**
an inclination sensor (34) is provided, said inclination sensor measuring the inclination of the device (1) relative to the surface of the earth and forwarding this measurement to the control unit (S), wherein the control unit (S) only activates the pump (12) after the actuating element (5) is actuated, when the measured inclination is within a predetermined inclination range.

2. The device as claimed in claim 1, wherein the fluid channel has a third channel portion (28) adjoining the second channel portion (27), said third channel portion extending as far as the outlet opening (29) and having a third cross-sectional surface which is larger than the second cross-sectional surface.

3. The device as claimed in claim 2, wherein the third channel portion (28) extends in a linear manner.

4. The device as claimed in claim 2 or 3, wherein the outlet nozzle (24) has a replaceable end cap (32) in which the third channel portion (28) is configured.

5. The device as claimed in one of claims 2 to 4, wherein the outlet nozzle (24) has at the outlet opening (29) a first region (37) with a first external diameter, wherein a second region (38) with a second diameter adjoins the first region (35) in the direction counter to the direction of the fluid, wherein the second diameter is smaller than the first diameter.

6. The device as claimed in one of the above claims, wherein the third cross-sectional surface of the third channel portion is smaller than the first cross-sectional surface of the first channel portion.

7. The device as claimed in one of the above claims, wherein the inner edge (31) of the outlet opening (29) is configured to be sharp-edged.

8. The device as claimed in one of the above claims, wherein the fluid channel of the outlet nozzle (24) is configured such that a linear through-flow is prevented.

9. The device as claimed in one of the above claims, wherein a non-return valve (30) is arranged in the fluid channel of the outlet nozzle (24), said non-return valve opening from a predetermined pressure of the fluid coming from the pump (12) and thus producing a fluidic connection of the pump (12) with the outlet opening (29).

10. The device as claimed in one of the above claims wherein the pump (12) is configured as a diaphragm pump.

11. The device as claimed in one of the above claims, wherein a plurality of pump cycles of the pump (12) are required in order to convey the volume of fluid for one droplet to be dispensed.

12. The device as claimed in one of the above claims, wherein a temperature measuring device (33) is provided, said temperature measuring device measuring the temperature of the fluid to be dispensed and forwarding this temperature to the control unit (S) which varies the activation of the pump (12) as a function of the measured temperature, in order to compensate for a temperature-induced volume change of the conveyed volume.

13. The device as claimed in one of the above claims, wherein the pump (12) has a motor and the control device (S), when the pump (12) is activated, detects when the motor of the pump (12) starts to rotate and from this time, after the defined time period for the operation of the pump (12) has elapsed, actively brakes the motor of the pump (12).

## Revendications

1. Dispositif de distribution goutte à goutte d'un fluide,
dans lequel le dispositif
un raccord de fluide (7), par lequel le fluide à distribuer peut être amené,
une pompe (12) qui est en communication fluidique avec le raccord de fluide (7),
une buse de sortie (24) qui est en communication fluidique avec la pompe (12),
un élément d'actionnement (5), et
une unité de commande (S) qui, lors de l'actionnement de l'élément d'actionnement (5), active la pompe (12) qui pompe le fluide acheminé vers la buse de sortie (24) pour le distribuer goutte à goutte,
la buse de sortie (24) possédant une ouverture de sortie (29) et un canal de fluide s'étendant jusqu'à l'ouverture de sortie, qui possède, dans le sens du fluide depuis la pompe (12) jusqu'à l'ouverture de sortie (29), une première section de canal (26) avec une première surface de section transversale et une deuxième section de canal (27) se raccordant à la première section de canal (26) avec une deuxième surface de section transversale,
la deuxième surface de section transversale étant plus petite que la première surface de section transversale,
**caractérisé en ce que**
il est prévu un capteur d'inclinaison (34) qui mesure l'inclinaison du dispositif (1) par rapport à la surface du sol et la transmet à l'unité de commande (S), l'unité de commande (S) n'activant la pompe (12) après actionnement de l'élément d'actionnement (5) que si l'inclinaison mesurée se situe dans une plage d'inclinaison prédéterminée.

2. Dispositif selon la revendication 1, dans lequel
le canal de fluide possède une troisième section de canal (28) qui se raccorde à la deuxième section de canal (27), qui s'étend jusqu'à l'ouverture de sortie (29) et qui possède une troisième surface de section transversale qui est plus grande que la deuxième surface de section transversale.

3. Dispositif selon la revendication 2, dans lequel
la troisième section de canal (28) s'étend de manière rectiligne.

4. Dispositif selon la revendication 2 ou 3, dans lequel
la buse de sortie (24) possède un embout interchangeable (32) dans lequel est formée la troisième portion de canal (28).

5. Dispositif selon l'une quelconque des revendications 2 à 4, dans lequel
la buse de sortie (24) possède, au niveau de l'ouverture de sortie (29), une première zone (37) ayant un premier diamètre extérieur, la première zone (35) étant suivie, dans la direction opposée à la direction du fluide, d'une deuxième zone (38) ayant un deuxième diamètre, le deuxième diamètre étant inférieur au premier diamètre.

6. Dispositif selon l'une des revendications précédentes, dans lequel
la troisième surface de section transversale de la troisième section de canal (28) est inférieure à la première surface de section transversale de la première section de canal (26).

7. Dispositif selon l'une quelconque des revendications précédentes, dans lequel le bord intérieur (31) de l'ouverture de sortie (29) est réalisé avec des arêtes vives.

8. Dispositif selon l'une des revendications précédentes, dans lequel
Le canal de fluide de la buse de sortie (24) est conçu de manière à empêcher un écoulement rectiligne.

9. Dispositif selon l'une des revendications précédentes, dans lequel
dans le canal de fluide de la buse de sortie (24) est disposé un clapet anti-retour (30) qui s'ouvre à partir d'une pression prédéterminée du fluide provenant de la pompe (12) et établit ainsi une communication de fluide de la pompe (12) avec l'orifice de sortie (29).

10. Dispositif selon l'une des revendications précédentes, dans lequel
la pompe (12) est conçue comme une pompe à membrane.

11. Dispositif selon l'une des revendications précédentes, dans lequel
plusieurs cycles de pompage de la pompe (12) sont nécessaires pour faire circuler le volume de fluide pour une goutte à distribuer.

12. Dispositif selon l'une des revendications précédentes, dans lequel
il est prévu un dispositif de mesure de la température (33) qui mesure la température du fluide à distribuer et la transmet à l'unité de commande (S) qui, en fonction de la température mesurée, fait varier la commande de la pompe (12) afin de compenser une variation de volume du volume transporté due à la température.

13. Dispositif selon l'une des revendications précédentes, dans lequel
la pompe (12) possède un moteur et le dispositif de commande (S) détecte, lors de l'activation de la pompe (12), le moment où le moteur de la pompe (12) commence à tourner et freine activement le moteur de la pompe (12) à partir de ce moment après l'écoulement de la période définie pour le fonctionnement de la pompe (12).
